# EUROPEAN PATENT SPECIFICATION

(11) **EP 3 419 605 B1**
(45) Date of publication and mention of the grant of the patent: **08.04.2026**
(21) Application number: 16706358.5
(22) Date of filing: 25.02.2016
(51) Int. Cl.: A61K 9/16, A61K 9/20, A61K 31/506, A61P 35/02

(54) **DASATINIB FORMULATION**
DASATINIB FORMULIERUNG
FORMULATION DE DASATINIB

(43) Date of publication of application: 02.01.2019
(73) Proprietor: Remedica Ltd, Limassol 3508 (CY); Pharmaceutical Oriented Services Ltd, 14452 Metamorfossi Attikis (GR)
(72) Inventor: PATTIHIS, Charalambos, Limassol 3508 (CY); NORDMANN, Antje, Limassol 3508 (CY); PANAGOPOULOS, Panagiotis, 14452 Metamorfossi Attikis (GR); PANITSAS, Konstantinos-Emmanouil, 14452 Metamorfossi Attikis (GR); ALEXAKI, Pandora, 14452 Metamorfossi Attikis (GR); KATAKALOU, Christina, 14452 Metamorfossi Attikis (GR); VIDALIS, Matthew, 14452 Metamorfossi Attikis (GR); KATHIOTOU, Georgia, 14452 Metamorfossi Attikis (GR)
(74) Representative: Isarpatent
(86) International application number: PCT/EP2016/054037
(87) International publication number: WO 2017/144109

(56) References cited:
- EP-A1- 2 359 813
- WO-A2-2006/121742
- CN-A- 102 836 159
- CN-A- 103 845 332
- B RASMITHA REDDY ET AL: "FORMULATION AND EVALUATION OF DASATINIB IMMEDIATE RELEASE TABLETS", WORLD JOURNAL OF PHARMACY AND PHARMACEUTICAL SCIENCES, January 2014 (2014-01-01), pages 1113 - 1123, XP055268539, Retrieved from the Internet <URL:http://www.wjpps.com/download/article/1396439797.pdf> [retrieved on 20160426]

## Description

The present invention relates to an oral dosage form comprising dasatinib, or a pharmaceutically acceptable salt thereof. In particular, the present invention relates to an oral dosage form comprising dasatinib, or a pharmaceutically acceptable salt thereof, wherein the amount of disintegrant present is controlled so as to provide desirable dissolution and / or pharmacokinetic profiles.

Dasatinib is commercially available under the trade mark Sprycel and is indicated for the treatment of adult patients with newly diagnosed Philadelphia chromosome positive (Ph+) chronic myelogenous leukaemia (C L) in the chronic phase; chronic, accelerated or blast phase CM L with resistance or intolerance to prior therapy including imatinib mesylate; and Ph+ acute lymphoblastic leukaemia (ALL) and lymphoid blast CML with resistance or intolerance to prior therapy.

Dasatinib was first described as a compound in the patent family that includes EP 1169038B. EP 1169038B generically and specifically claims dasatinib. Compositions forthe compounds of EP 1169038B are generally described in paragraphs [0075] to [0080], but no specific compositions for dasatinib are disclosed.

Later patent specifications do disclose specific compositions for dasatinib. For example, WO 2006/121742 describes specific film coated tablets of dasatinib. In particular, the film coating is selected so as to be non-reactive and not to cause decomposition of dasatinib. WO 2006/121742 describes the various other excipients that could be used in the compositions thereof, and specific Examples are also provided. Specifically, the Examples describe tablets of varying strengths of dasatinib (from 5 to 150 mg). The excipients used in each tablet are lactose monohydrate, microcrystalline cellulose, hydroxypropylcellulose, croscarmellose sodium and magnesium stearate, and Opadry white is provided as the film coating.

Given the differing strengths of the dasatinib tablets as used in the Examples of WO 2006/121742, different amounts of excipients were also used. As disintegrant, croscarmellose sodium was used, and this was present at the following amounts - 4.0 mg (Examples I to III), 3.2 mg (Example IV), 8.0 mg (Example V), 11.2 mg (Example VI), and 24.0 mg (Example VII). Based on the amount of dasatinib present, the above disintegrant amounts are present at the following ratios to the amount of dasatinib - 0.8 : 1.0 (Example I), 0.08 : 1.0 (Example II), 0.16 : 1.0 (Examples III to VII).

WO 2006/121742 also describes the impact of dasatinib particle size and / or intra / extra granular location thereof, on compressibility. More specifically, page 8 of WO 2006/121742 describes that a formulation using larger particle size of dasatinib (D90 - 120 µm (microns)) was found to have poor compression properties when all of microcrystalline cellulose was added intragranularly. Conversely according to WO 2006/121742, the formulation using a particle size of dasatinib with D90 up to about 130 µm (microns) was found to have acceptable compression properties when a portion of the microcrystalline cellulose was added in the extragranular phase. Alternatively according to WO 2006/121742, the particle size of dasatinib may be up to or equal to 150 µm (microns). To some extent however, this information is not particularly helpful, given no disclosure was provided as to the particle size measuring techniques employed. As a skilled person will immediately realize, different particle size measurements are obtained for any given sample, depending on the measurement technique that is used. Still further, no meaningful analysis can be performed of the dissolution properties and as such associated bioavailability of the specific tablets of the Examples of WO 2006/121742, because there is no disclosure whatsoever in these Examples as to the particle size of the specific Form of dasatinib that was used and the associated measurement technique.

CN 103845332 A provides a dasatinib pharmaceutical composition directly using dasatinib anhydrate.

A skilled reader would also be aware that the presence and associated amounts of specific excipients has an effect on the dissolution of an active pharmaceutical ingredient as present in a dosage form. Furthermore, the skilled reader would also be aware that particle size of an active pharmaceutical ingredient would also have an effect on the dissolution of an active pharmaceutical ingredient as present in a dosage form. For excipients, for example an increase in an amount of disintegrant present would be expected to enhance dissolution of an active pharmaceutical ingredient as present in the dosage form. For particle size, it would be expected that a decrease in particle size would also enhance dissolution of an active pharmaceutical ingredient as present in the dosage form. We have however now found that contrary to general understanding, that when the disintegrant content of an oral dosage form of dasatinib is increased within a specific range, then the dissolution of dasatinib from the dosage form generally decreases and as such disintegrant content can be used in an unexpected manner to control the dissolution rate of dasatinib from the dosage form.

According to the present invention therefore, there is provided an oral dosage form comprising dasatinib, or a pharmaceutically acceptable salt thereof, together with at least one disintegrant, wherein the ratio of the amount of disintegrant to the amount of dasatinib, or a pharmaceutically acceptable salt thereof, is in the range of 0.3 : 1.0 to 1.0 : 1.0, based on the amount of dasatinib free base as present in the dosage form, and wherein said dasatinib has a D90 in the range of 50 to 100 µm (microns) when measured by laser diffraction techniques, wherein the disintegrant comprises croscarmellose sodium.

Preferably, dasatinib as present in an oral dosage form according to the present invention as described above has a particle size distribution, wherein D90 is in the range of 50 to 90 µm (microns), preferably 50 to 80 µm (microns), more preferably 50 to 70 µm (microns), even more preferably 50 to 60 µm (microns), and most preferably a D90 of about 55 µm (microns). The particle size distribution was measured by laser diffraction techniques typically employing a mastersizer 2000 laser particle size analyzer.

Furthermore, the oral dosage form according to the present invention has a dissolution profile wherein about 45 to 70% of dasatinib is released in 60 minutes, when tested in a USP Type-II Paddle apparatus, wherein the dissolution medium comprises 900 mL of an acetate buffer having a pH of 4.5, at 50 rpm, at a temperature of 37°C ± 0.5°C. The acetate buffer is preferably prepared by dissolving an acetate salt, such as sodium acetate, in water, and the resulting solution is either added to, or to which solution is added, acetic acid.

Furthermore, it is preferred that an oral dosage form according to the present invention provides a maximum measured plasma dasatinib concentration over time (Cmax) ratio with respect to the reference product Sprycel in the range of 0.80 to 1.25. It is also preferred that an oral dosage form according to the present invention provides an AUC ratio with respect to the reference product Sprycel in the range of 0.80 to 1.25 as calculated by the linear trapezoidal method, up to the last observed value. As referenced herein, the reference product Sprycel is a film coated dasatinib (140 mg) tablet, having the following excipients: lactose monohydrate, microcrystalline cellulose, croscarmellose sodium, hydroxypropyl cellulose and magnesium stearate. The film coating comprises hypromellose, titanium dioxide and acrogol 400. As a skilled reader will be aware, the Sprycel product is a commercially available product.

A particularly preferred ratio of disintegrant to dasatinib to be used in an oral dosage form according to the present invention is in the range of 0.3 : 1.0 to 0.5 : 1.0, based on the amount of dasatinib free base as present in the dosage form.

The disintegrant to be used in accordance with the present invention is croscarmellose sodium.

Dasatinib as used in the present invention can be dasatinib free base or a pharmaceutically acceptable salt thereof. The dasatinib can be anhydrous or present as a hydrated Form, such as dasatinib monohydrate. Typically the dasatinib is present as an anhydrous free base Form, and even more preferably dasatinib anhydrous N6 polymorph can be a preferred polymorphic Form for use in the present invention. As already documented in the patent literature, for example WO 2005/077945, dasatinib anhydrous N6 polymorph can be characterized by the following representative 2theta values: 6.8, 11.1, 12.3, 13.2, 13.7, 16.7, 21.0, 24.3 and 24.8 ± 0.2° 2theta. Such X PD measurement can be carried out based on the skill and common general knowledge of one of ordinary skill in the art and indeed above referenced WO 2005/077945 provides teaching as to the techniques and apparatus used.

Compositions intended for oral use according to the present invention may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Oral dosage forms to be used in the present invention can contain the active ingredient dasatinib in admixture with non-toxic pharmaceutically acceptable excipients which are suitable for the manufacture of tablets. These excipients may be for example, inert diluents, such as microcrystalline cellulose, lactose, calcium carbonate, sodium carbonate, calcium phosphate or sodium phosphate; binding agents, for example hydroxypropyl cellulose, starch, gelatin, polyvinylpyrrolidone and polyethylene glycol; and lubricating agents, for example, magnesium stearate, stearic acid or talc. A disintegrant to be used in an oral dosage form according to the present invention is as hereinbefore described.

According to the present invention, croscarmellose sodium is used as a disintegrant; microcrystalline cellulose and lactose monohydrate are preferably used as inert diluents or "fillers"; hydroxypropyl cellulose is preferably used as a binder; and magnesium stearate is preferably used as a lubricant. Preferred specific compositions according to the present invention and employing the above excipients are hereinafter described in the specific Examples.

The oral dosage forms of the present invention can be tablets.

The tablets may be uncoated or they may be coated by known techniques. Preferably the tablets are film coated. More preferably, the coating material used is a polyethylene glycol (PEC) free moisture protective coating material (for example, Opadry 200). For example, an Opadry coating as illustrated in the Examples is used for an oral dosage form according to the present invention.

An oral dosage form according to the present invention exhibits chemical and polymorphic stability after 6 months of storage under accelerated conditions 40°C/75% H when packed in aluminium-aluminium blisters or high density polyethylene bottles. Such stability is further illustrated herein by the accompanying Examples and Figures and in particular as illustrated herein when tablets according to the present invention are subjected to 6 months of storage under accelerated conditions 40°C/75%RH when packed in high density polyethylene bottles then substantially no polymorphic interconversion was observed.

In a particularly preferred embodiment, an oral dosage form according to the present invention is prepared by a wet granulation process. More preferably, the coating material used is a PEC free moisture protective coating material (for example, Opadry 200).

There is also described but not claimed a method of providing a dasatinib oral dosage form having a dissolution profile and / or a pharmacokinetic profile as hereinbefore described, which method comprises including a disintegrant in an amount in the oral dosage form whereby the ratio of the amount of disintegrant to the amount of dasatinib, or a pharmaceutically acceptable salt thereof, is in the range of 0.3 : 1.0 to 1.0 : 1.0 based on the amount of dasatinib free base as present in the dosage form. There is also described but not claimed use of a disintegrant for controlling (lowering) a dissolution profile and / or a pharmacokinetic profile of an oral dosage form comprising dasatinib as hereinbefore described, wherein said disintegrant is present in an amount in the oral dosage form whereby the ratio of the amount of disintegrant to the amount of dasatinib, or a pharmaceutically acceptable salt thereof, is in the range of 0.3 : 1.0 to 1.0 : 1.0 based on the amount of dasatinib free base as present in the dosage form. More preferred ratios for the above methods and uses, are wherein the ratio of the amount of disintegrant to the amount of dasatinib, or a pharmaceutically acceptable salt thereof, is in the range of 0.3 : 1.0 to 0.5 : 1.0, based on the amount of dasatinib free base as present in the dosage form.

The present invention also provides an oral dosage form as herein before described, for use in the treatment of leukemia, and specifically for use in the treatment of newly diagnosed Philadelphia chromosome positive (Ph+) chronic myelogenous leukaemia (CM L) in the chronic phase; chronic, accelerated or blast phase CM L with resistance or intolerance to prior therapy including imatinib mesylate; and Ph+ acute lymphoblastic leukaemia (ALL) and lymphoid blast C L with resistance or intolerance to prior therapy.

The present invention is now illustrated by the following Examples, which do not limit the scope of the invention in any way.

### Example 1:

A tablet was prepared with a disintegrant : API ratio of 0.5 : 1.0 based on the following recipe:

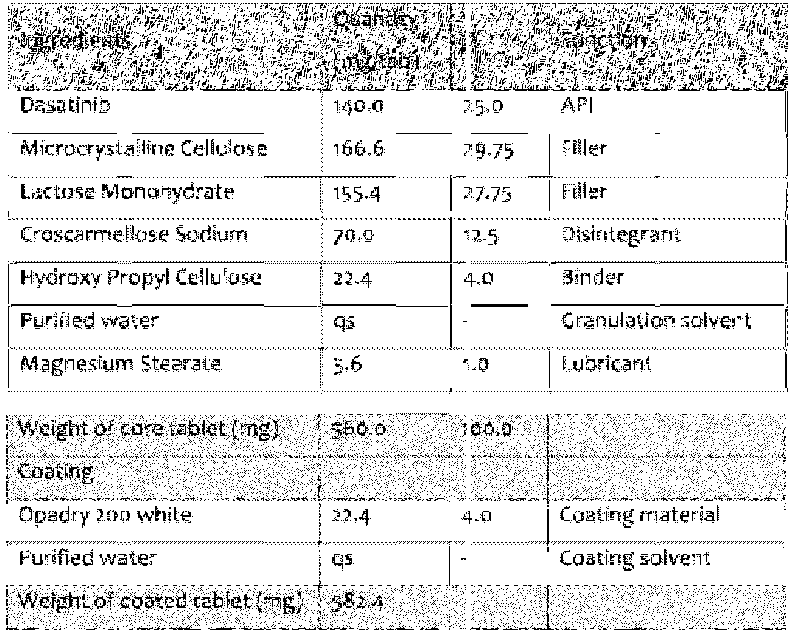

### Manufacturing process steps:

1) Materials were loaded and dry mixed together in a high shear mixer granulator.
2) Hydroxy Propyl Cellulose (binder solution) was dissolved in purified water by stirring.
3) The solution of Step 2 was added in the dry mixed material. Kneading of the mass takes place, until a desired consistency of wet mass was achieved.
4) The wet mass of Step 3, was wet-sized through screen.
5) The sized, wet granules of Step 4 were dried, until %LOD settles between 1.5 and 3 w/w.
6) The dried granules of Step 5 were further dry-sized.
7) The sized granules of Step 6 were blended with croscarmellose sodium and lubricated with magnesium stearate.
8) The blend of Step 7 was compressed into round shaped, concave tablets.
9) The obtained core tablets of Step 8, were film coated using Opadry 200, white.

### Reference Example 2

A tablet was prepared with a disintegrant : API ratio of 0.2 : 1.0 based on the following recipe:

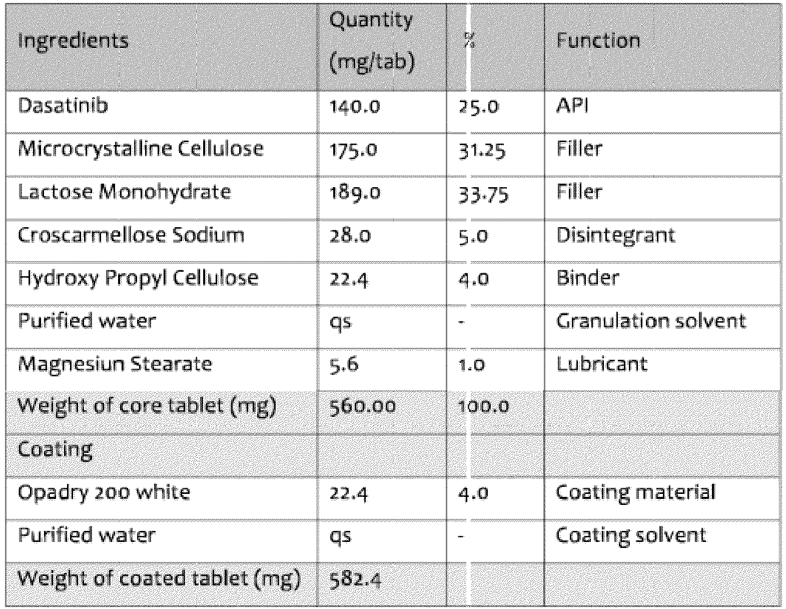

### Manufacturing process steps:

These were carried out in accordance with the above described process of Example 1.

### Example 3: Dissolution results:

The following dissolution results were obtained for tablets with excipients as described in Example 1 and Reference Example 2, but with the following disintegrant / API ratios. Furthermore, the Cmax and AUC values that are referenced are ratios of the respective values for such tablets according to the present invention versus the Cmax and AUC values for the Sprycel product similarly including 140 mg of dasatinib.

| **Disintegrant: API ratio** | **Time point** | **0** | **5** | **10** | **15** | **20** | **30** | **45** | **60** | **Cmax** | **AUC** |
|---|---|---|---|---|---|---|---|---|---|---|---|
| **0.2:1** | **Mean (%)** | **0** | **50.5** | **67.2** | **72.9** | **75.8** | **78.1** | **78.5** | **79.0** | **1.16** | **1.29** |
| | **% RSD** | 0 | 5.4 | 2.1 | 3.2 | 3.8 | 4.0 | 4.4 | 4.8 | | - |
| **0.3:1** | **Mean (%)** | **0** | **33.6** | **43-5** | **48.8** | **53.0** | **57.4** | **62.4** | **65.9** | **1.07** | **1.25** |
| | **% RSD** | 0 | 4.0 | 4.7 | 4.7 | 4.7 | 5.0 | 5.0 | 5.5 | - | - |
| **0.5:1** | **Mean (%)** | **0** | **21.0** | **32.7** | **38.1** | **40.5** | **43.4** | **45.8** | **48.2** | **0.83** | **1.08** |
| | **% RSD** | 0 | 1.5 | 4.0 | 0.3 | 1.0 | 0.5 | 1.0 | 1.1 | - | - |
| **Sprycel 0.16:1** | **Mean (%)** | **0** | **20.9** | **25.2** | **27.1** | **29.4** | **31.8** | **33.7** | **34.8** | **-** | - |
| | **% RSD** | 0 | 15.8 | 6.4 | 2.3 | 3.6 | 3.0 | 1.9 | 2.0 | - | - |

### Dissolution method:

| Dissolution parameters: | |
|---|---|
| Apparatus | USP Type-II, Paddle |
| Dissollution medium | pH 4.5 acetate buffer |
| Volume | 900 mL |
| Rotations Per Minute | 50 |
| Temperature | 37°C ± 0.5°C |
| Sampling Intervals | 5, 10, 15, 20,30,45, 60 min and recovery (10 minutes at 200rpm) |
| Dissolution medium preparation: Acetate Buffer pH 4.5 | Dissolve 2.99 g of Sodium acetate trihydrate in 800mL water. Add 14.0mL of 2 M acetic acid and dilute to 1000 mL with water, mix. |

### Example 4: Stability on storage

| **DASATINIB F.C TABS - STABILITY REPORT** | | | |
|---|---|---|---|
| **Product name** | Dasatinib film coated tablets | **Stability condition** | 40°C ± 2°C / 75% RH ± 5% RH |
| **Strength** | 140 mg | **Packing details** | 75 cc HDPE bottles, |
| **Batch size** | 6500 tablets | | |

| **Analytical Tests** | **Initial** | **6^{th} month** |
|---|---|---|
| **Related Substances** | % | % |
| **Impurity A** | 0.01 | 0.02 |
| **Impurity B** | 0.01 | 0.01 |
| **Impurity C** | 0.02 | 0.03 |
| **Impurity D** | 0.01 | ND |
| **Any unspecified impurity** | 0.01 | 0.01 |
| **Total Impurities** | 0.06 | 0.07 |
| **Assay** | 102.1 | 97.6 |
| **XRD** | No transformation | No transformation |

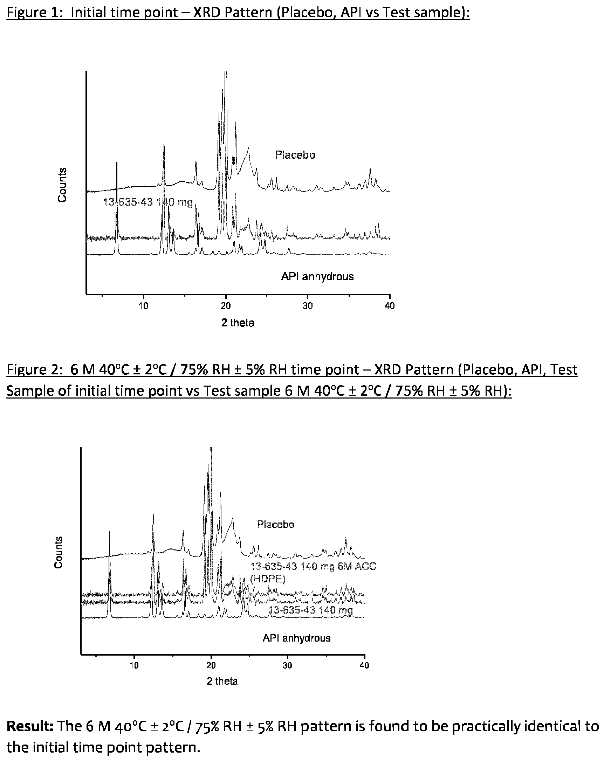

140 mg tablets according to the present invention were prepared, packaged in HDPE bottles and chemical and polymorphic stability was investigated further to 6 month storage at 40°C ± 2°C / 75% RH ± 5% RH. From the above results, it can be seen that both chemical and polymorphic stability was retained during such 6 month storage. Figures 1 and 2 also show in further detail the observed polymorphic stability.

## Claims

1. An oral dosage form comprising dasatinib, or a pharmaceutically acceptable salt thereof, together with at least one disintegrant, wherein the ratio of the amount of disintegrant to the amount of dasatinib, or a pharmaceutically acceptable salt thereof, is in the range of 0.3 : 1.0 to 1.0 : 1.0 based on the amount of dasatinib free base as present in the dosage form, and wherein said dasatinib has a D90 in the range of 50 to 100 µm (microns) when measured by laser diffraction techniques, wherein the disintegrant comprises croscarmellose sodium.

2. A dosage form according to claim 1, wherein said dasatinib has a D90 in the range of 50 to 90 µm (microns) when measured by laser diffraction techniques, preferably a D90 in the range of 50 to 80 µm (microns), more preferably a D90 in the range of 50 to 70 µm (microns), even more preferably a D90 in the range of 50 to 60 µm (microns) and most preferably a D90 of about 55 µm (microns).

3. A dosage form according to claim 2, wherein the following excipients are also present: microcrystalline cellulose and lactose monohydrate as inert diluents; hydroxypropyl cellulose as a binder; and magnesium stearate as a lubricant.

4. A dosage form according to any preceding claim, which is a tablet.

5. A tablet according to claim 4, which is film coated, preferably wherein said film coating comprises a polyethylene glycol free moisture protective coating material.

6. A dosage form according to any of the preceding claims, wherein said dasatinib comprises anhydrous N6 polymorph.

7. A dosage form according to any preceding claim, which is prepared by a wet granulation process.

8. A dosage form according to any of claims 1 to 7, for use in the treatment of leukemia.

9. A dosage form according to claim 8, for use in the treatment of newly diagnosed Philadelphia chromosome positive (Ph+) chronic myelogenous leukaemia (CML) in the chronic phase; chronic, accelerated or blast phase CML with resistance or intolerance to prior therapy including imatinib mesylate; and Ph+ acute lymphoblastic leukaemia (ALL) and lymphoid blast CML with resistance or intolerance to prior therapy.

## Patentansprüche

1. Orale Dosierungsform, umfassend Dasatinib oder ein pharmazeutisch annehmbares Salz davon zusammen mit mindestens einem Sprengmittel, wobei das Verhältnis der Menge an Sprengmittel zu der Menge an Dasatinib oder einem pharmazeutisch annehmbaren Salz davon im Bereich von 0,3 : 1,0 bis 1,0 : 1,0 liegt, bezogen auf die Menge an freier Dasatinib-Base, wie sie in der Dosierungsform vorliegt, und wobei das Dasatinib einen D90 im Bereich von 50 bis 100 µm (Mikrometer) aufweist, gemessen durch Laserbeugungstechniken, wobei das Sprengmittel Croscarmellose-Natrium umfasst.

2. Dosierungsform nach Anspruch 1, wobei das Dasatinib einen D90 im Bereich von 50 bis 90 µm (Mikrometer) aufweist, gemessen durch Laserbeugungstechniken, vorzugsweise einen D90 im Bereich von 50 bis 80 µm (Mikrometer), mehr bevorzugt einen D90 im Bereich von 50 bis 70 µm (Mikrometer), noch mehr bevorzugt einen D90 im Bereich von 50 bis 60 µm (Mikrometer) und am meisten bevorzugt einen D90 von etwa 55 µm (Mikrometer).

3. Dosierungsform nach Anspruch 2, wobei die folgenden Hilfsstoffe ebenfalls vorhanden sind: mikrokristalline Cellulose und Lactosemonohydrat als inerte Verdünnungsmittel; Hydroxypropylcellulose als Bindemittel; und Magnesiumstearat als Gleitmittel.

4. Dosierungsform nach einem der vorhergehenden Ansprüche, die eine Tablette ist.

5. Tablette nach Anspruch 4, die filmbeschichtet ist, wobei die Filmbeschichtung vorzugsweise ein Polyethylenglycol-freies feuchtigkeitsschützendes Beschichtungsmaterial umfasst.

6. Dosierungsform nach einem der vorhergehenden Ansprüche, wobei das Dasatinib wasserfreies N6-Polymorph umfasst.

7. Dosierungsform nach einem der vorhergehenden Ansprüche, die durch ein Nassgranulationsverfahren hergestellt wird.

8. Dosierungsform nach einem der Ansprüche 1 bis 7 zur Verwendung bei der Behandlung von Leukämie.

9. Dosierungsform nach Anspruch 8 zur Verwendung bei der Behandlung von neu diagnostizierter Philadelphia-Chromosom-positiver (Ph+) chronischer myelogener Leukämie (CML) in der chronischen Phase; chronischer, beschleunigter oder Blast-Phasen-CML mit Resistenz oder Intoleranz gegenüber einer früheren Therapie, einschließlich Imatinib-Mesylat; und Ph+ akuter lymphoblastischer Leukämie (ALL) und Lymphoid-Blast-CML mit Resistenz oder Intoleranz gegenüber einer früheren Therapie.

## Revendications

1. Forme posologique orale comprenant du dasatinib ou un sel pharmaceutiquement acceptable de celui-ci, ainsi qu'au moins un agent désagrégeant, le rapport entre la quantité de l'agent désagrégeant et la quantité de dasatinib ou d'un sel pharmaceutiquement acceptable de celui-ci étant compris entre 0,3 : 1,0 et 1,0 : 1,0, sur la base de la quantité de base libre de dasatinib présente dans la forme posologique, et le dasatinib présentant une valeur D90 comprise entre 50 et 100 µm (micromètres), mesurée par des techniques de diffraction laser, l'agent désagrégeant comprenant de la croscarmellose sodique.

2. Forme posologique selon la revendication 1, dans laquelle le dasatinib présente une valeur D90 comprise entre 50 et 90 µm (micromètres), mesurée par des techniques de diffraction laser, de préférence une valeur D90 comprise entre 50 et 80 µm (micromètres), plus préférentiellement une valeur D90 comprise entre 50 et 70 µm (micromètres), encore plus préférentiellement une valeur D90 comprise entre 50 et 60 µm (micromètres), et très préférentiellement une valeur D90 d'environ 55 µm (micromètres).

3. Forme posologique selon la revendication 2, dans laquelle les excipients suivants sont également présents : cellulose microcristalline et lactose monohydraté en tant qu'excipients diluants inertes ; hydroxypropylcellulose en tant qu'agent liant ; et stéarate de magnésium en tant qu'agent lubrifiant.

4. Forme posologique selon l'une quelconque des revendications précédentes, qui est un comprimé.

5. Comprimé selon la revendication 4, qui est pelliculé, de préférence dans lequel le pelliculage comprend un matériau d'enrobage protecteur contre l'humidité exempt de polyéthylène-glycol.

6. Forme posologique selon l'une quelconque des revendications précédentes, dans laquelle le dasatinib comprend le polymorphe N6 anhydre.

7. Forme posologique selon l'une quelconque des revendications précédentes, préparée par un procédé de granulation humide.

8. Forme posologique selon l'une quelconque des revendications 1 à 7, pour une utilisation dans le traitement de la leucémie.

9. Forme posologique selon la revendication 8, pour une utilisation dans le traitement de la leucémie myéloïde chronique (LMC) positive au chromosome Philadelphie (Ph+) nouvellement diagnostiquée en phase chronique ; de la LMC en phase chronique, accélérée ou blastique présentant une résistance ou une intolérance à un traitement antérieur comprenant le mésylate d'imatinib ; ainsi que de la leucémie lymphoblastique aiguë (LLA) Ph+ et de la LMC lymphoïde blastique présentant une résistance ou une intolérance à un traitement antérieur.
